(19) Europäisches Patentamt
European Patent Office
Office européen des brevets

(11) Publication number: 0 119 359 B1

(12) EUROPEAN PATENT SPECIFICATION

(45) Date of publication of patent specification: 10.06.87

(51) Int. Cl.⁴: C 07 C 2/58, C 07 C 5/27, C 07 C 53/138, C 07 C 143/20

(21) Application number: 83307851.2

(22) Date of filing: 22.12.83

# (54) Adamantyl carboxylic and sulfonic acid catalyzed paraffin-olefin alkylation and paraffin isomerization.

(30) Priority: 15.03.83 US 475450
15.03.83 US 475451

(43) Date of publication of application:
26.09.84 Bulletin 84/39

(45) Publication of the grant of the patent:
10.06.87 Bulletin 87/24

(84) Designated Contracting States:
BE DE FR GB IT NL

(56) References cited:
DE-A-2 064 904
DE-A-2 358 469
DE-A-2 358 499
US-A-3 231 633
US-A-3 655 807
US-A-3 671 598
US-A-3 707 576
US-A-4 229 611
US-A-4 357 481
US-A-4 357 482
US-A-4 357 483
US-A-4 357 484

(73) Proprietor: Exxon Research and Engineering Company
P.O.Box 390 180 Park Avenue
Florham Park New Jersey 07932 (US)

(72) Inventor: Kramer, George M.
36 Arden Court
Berkeley Heights New Jersey, 07922 (US)

(74) Representative: Pitkin, Robert Wilfred et al
ESSO Engineering (Europe) Ltd. Patents & Licences Apex Tower High Street
New Malden Surrey KT3 4DJ (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 119 359 B1

**Description**

This invention relates to a catalytic process for producing branched paraffins under strong acid catalyzed conditions in the presence of adamantyl carboxylic and sulfonic acids as hydride transfer catalysts.

Alkylation of olefins by carbonium ions or isomerization of paraffins under strong acid conditions are well-known processes for producing a wide variety of useful hydrocarbon materials and particularly gasoline additives. For example, 2,2,4-trimethylpentane is a common blending agent used for gasoline octane improvement which can be produced by alkylating isobutylene with isobutane in sulfuric acid or liquid HF.

An example of an acid-catalyzed reaction process is described in U.S. Patent No. 3,231,633.

Hydrocarbon conversion processes employing novel Lewis acid systems are disclosed in U.S. Patent No. 4,229,611 and U.S. Patent No. 4,162,233, both assigned to Exxon Research and Engineering Company.

U.S. Patent No. 3,671,598 describes a process for isomerizing saturated cyclic hydrocarbons under strong acid conditions in the presence of an adamantane hydrocarbon. However, no suggestion is made that a specifically substituted adamantane compound might be useful in paraffin-olefin alkylation under strong acid conditions or that specifically substituted adamantanes, particularly those with carboxylic acid or sulfonic acid substituents, might be more effective in increasing the rate of isomerization of paraffins to branched isomers.

U.S. Patents 4,357,481; 4,357,484; 4,357,482; and 4,357,483 to George M. Kramer (all issued November 2, 1982, and assigned to Exxon Research and Engineering Company) disclose the use of adamantane hydrocarbons in paraffin-olefin alkylation and non-cyclic paraffin isomerization, and the use of aminoalkyladamantanes in paraffin-olefin alkylation and paraffin isomerization, respectively, in which rates of reaction are substantially increased as compared to those obtained in the absence of the specifically disclosed adamantane. However, none of the patents disclose or suggest the use of carboxy- or sulfoxy-containing adamantanes as rate enhancing agents in alkylation or isomerization processes.

New methods for producing such branched paraffinic hydrocarbons useful as octane improvement agents are constantly being searched for in an effort to improve isomerization and paraffin-olefin alkylation process efficiencies. More active catalysts enable these rearrangements to be conducted at lower temperatures where thermodynamic equilibria are more favorable to branched structures, an important factor in butane, pentane and hexane isomerization.

It has been found that the presence of an adamantyl carboxylic acid or sulfonic acid in a strong acid system containing a paraffinic hydrocarbon, capable of participating in hydride transfer or isomerization, rapidly increases the rate of a paraffin-olefin alkylation reaction, or of the isomerization of said paraffinic hydrocarbon, presumably through increased intermolecular hydride transfer that the paraffin undergoes in the system. In the production of octane-increasing agents, this should lead to the utilization of smaller and more efficient reactors, which enhances the economics of the process.

More specifically, by this invention, there is provided a process comprising contacting a $C_4$—$C_6$ paraffinic hydrocarbon with a strong acid system and in the presence of an adamantyl carboxylic acid or sulfonic acid, containing at least one unsubstituted bridgehead position, at a temperature of −100 to 150°C, thereby producing a branched paraffinic hydrocarbon where only the $C_4$—$C_6$ paraffinic hydrocarbon is used as the feed to the process, a branched isomer of said hydrocarbon having the same number of carbon atoms is produced. In that embodiment of the invention where a $C_2$—$C_5$ olefin is present with the $C_4$—$C_6$ paraffinic hydrocarbon in the reactor, the product of the resulting paraffin-olefin alkylation process will be a $C_6$—$C_{11}$ branched paraffinic hydrocarbon.

In the process, the total described range of applicable paraffins and olefins (for alkylation) can be used in the present process, under very strong acid conditions, e.g. $AlBr_3$. However, in the slightly weaker acid systems, such as $H_2SO_4$ and HF, n-paraffins like n-butane do not generally undergo the isomerization process and combinations like n-butane and ethylene do not generally undergo the alkylation process since they require the stronger acid systems, as described herein.

There is also provided by this invention compositions of matter of the formula:

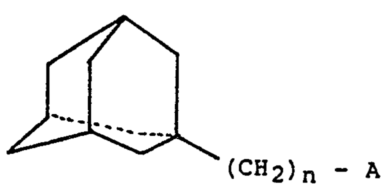

wherein A is COOH or $SO_3H$, and n is 0—16, with the proviso that where A is COOH, n is 8—16, and wherein the adamantane ring and alkylene chain can be substituted with substituents which are inert or unreactive under process conditions for producing branched paraffinic hydrocarbons.

The reason that an adamantyl carboxylic acid or sulfonic acid serves to increase the rate of intermolecular hydride transfer during branched paraffin isomerization or during paraffin-olefin alkylation is not clearly understood. One theory that we do not wish to be bound by is that reversible hydride transfer

from the bridgehead position of the adamantyl group to a carbonium ion in solution is enhanced due to lack of steric repulsions in the transition state involving the adamantyl group, as compared to hydride transfer involving a paraffinic hydrocarbon and the same carbonium ion. Since intermolecular hydride transfer is generally the rate determining step in paraffin-olefin alkylation and in paraffin isomerization (see "Industrial Laboratory Alkylation", edited by Lyle F. Allbright and Arthur R. Goldsby, ACS Symposium Series 55, Published Washington, D.C., 1977, Chapter One, "Alkylation Studies" by G. M. Kramer), then the presence of the adamantyl carboxylic acid or sulfonic acid will serve to significantly increase the reaction rate of these processes.

In the process, $C_4$—$C_6$ paraffinic hydrocarbons are isomerized or $C_2$—$C_5$ olefins are alkylated by $C_4$—$C_6$ linear or branched paraffinic compounds. As is well-known, the extent of the rearrangement and the possibility of changing the degree of branching of the paraffin as distinct from the possibility of inducing an alkyl shift, depends primarily on the acid system. The adamantyl carboxylic acid or sulfonic acid compound catalyzes the process appropriate to the acid employed. Examples of operable paraffins include n-butane, isobutane, isopentane, n-pentane, 2-methylpentane, 3-methylpentane, n-hexane and mixtures thereof.

In the isomerization process embodiment of the invention, preferred paraffins in the process are 2- and 3-methylpentane, n-hexane, n-pentane and n-butane, or refinery streams containing mixtures of these components.

The product paraffins of the isomerization embodiment of the process are $C_4$—$C_6$ branched paraffinic hydrocarbons. Representative examples include isobutane, isopentane, 2-methylpentane, 3-methylpentane, 2,3-dimethyl butane, 2,2-dimethylbutane. The preferred product paraffinic hydrocarbons in the process are the most highly branched isomers in each of the $C_4$, $C_5$ and $C_6$ product streams. The product paraffins are useful as gasoline blending agents for octane improvement and/or hydrocarbon solvents.

In the paraffin-olefin alkylation process embodiment of the invention, it is preferred that the starting paraffinic compound used is branched, since branching facilitates reaction and results in a higher octane number product for internal combustion engine purposes. A preferred paraffin hydrocarbon in this embodiment of the process is isobutane.

Linear or branched $C_2$—$C_5$ olefins useful in the alkylation embodiment of the process include ethylene, propylene, butene-1, cis or trans-butene-2, isobutylene, pentene-1, pentene-2, methylbutenes and mixtures thereof. Preferred olefins are butylenes.

The weight ratio of paraffin to olefin used in the process is generally 3—20 to 1 and preferably about 10 to 1. The olefin space velocity generally used in the alkylation is in the range of 0.01 to 1 liquid volume olefin/liquid volume of acid/hour. A preferred embodiment of the process is where the olefin and paraffin are co-introduced into the strong acid system as a feedstream mixture.

The product hydrocarbons in the reaction of isobutane with butylenes are mainly $C_8$ branched paraffins. Representative examples include 2,2,4-, 2,3,4-, 2,3,3- and 2,2,3-trimethylpentanes, 2,4-,2,3- and 2,5-dimethylhexanes. Preferred products in the alkylation process are the trimethylpentanes.

Carbonium ions in the process can be generated in various ways; most often by protonation of an olefin, but also by oxidation of a paraffin or in situ from their respective halides, e.g., t-butyl chloride, in the acid system, or they can be generated from the free hydrocarbon by undergoing intermolecular hydride transfer with in situ generated adamantyl cation. The preferred method depends on the acid system, but in $H_2SO_4$ or HF, they are readily formed by protonation of olefins.

The phrase "a strong acid system", as used herein, refers to the acid system capable of assisting in generating carbonium ions in the process and includes an "acid component" and a solvent, or one material that can function in both capacities, such as concentrated sulfuric acid or liquid HF. The acid system can be solid, liquid, or in the vapor phase. Preferably, the acid system is a liquid.

The strong acid components in the acid system are conventional protic, aprotic, or Lewis acids and include $AlBr_3$, $AlCl_3$, $GaCl_3$, $TaF_5$, $SbF_5$, $AsF_5$, $BF_3$, HF, HCl, HBr, $H_2SO_4$, $HSO_3F$, $CF_3SO_3H$ and mixtures thereof. Preferred acid components in the process, when aimed at preparing most highly branched products, are $AlBr_3$, $GaCl_3$, $H_2SO_4$ or $TaF_5$ with $H_2SO_4$ generally being the most preferred because of cost, availability and other desired processing properties. If a rapid but limited rearrangement is desired, $H_2SO_4$ or HF would be the preferred acids. An example of the former is the isomerization of n-hexane to dimethylbutanes and an example of the latter is the isomerization of 2-methylpentane to 3-methylpentane. Also, HCl and HBr are preferably not used alone, but are used in combination with other Lewis acids, e.g., $AlCl_3$ or $AlBr_3$.

Also a component of the "acid system", if required, is a solvent for the acid component. For Lewis acids, halogenated paraffins and aromatics are generally used; representative examples include $CH_3Br$, $CH_2Br_2$, $CH_2Cl_2$, 1,2-dichloroethane, 1,2,3-trichlorobenzene, 1,2,3,4-tetrachlorobenzene, pentafluorobenzene, HF, $H_2SO_4$, $CF_3SO_3H$, $HSO_3F$ and the like, and mixtures thereof.

The molar concentration of acid component in the solvent, if they are different materials, is generally between 0.1 and 8.0M, and preferably 0.5 to 4.0M (moles/liter).

The volume ratio of the acid system to the paraffinic hydrocarbon to be isomerized is generally 5/1 to 1/5, and preferably 3/1 to 1/3 parts by volume. However, larger and smaller ratios can be effectively employed.

The adamantyl acid useful in the process contains at least one carboxy or sulfoxy group, preferably being an alkylcarboxy or alkylsulfoxy group, and at least one unsubstituted adamantyl bridgehead

position, is preferably surface active, and can be prepared by conventional methods in the art. (See, for example, J. Med. Chem. 1967, Vol. 10, pp 517—521 for synthesis of carboxylic acid derivatives, when n in the following formula is 2—6 which procedures, i.e., Arndt-Eistert and malonic ester syntheses, will also be applicable for the corresponding higher acid derivatives of n being 7—16. Syntheses of the corresponding sulfonic acid deviations can be accomplished for example by reacting the appropriate adamantyl- or adamantylalkylbromide with sodium sulfite or bisulfite. The adamantylalkylbromide can be produced for example from the corresponding carboxylic acid by reduction to the alcohol followed by the reaction with hydrogen bromide. By the term "surface active", is meant that the adamantyl acid depresses the surface tension of the acid system, and promotes the formation of an emulsion between the acid phase and hydrocarbon phase when used at low concentration, typically in the range of $10^{-2}$ to $10^{-1}$ moles/liter, based on the liquid acid layer.

The adamantyl acid useful in the process is preferably of the formula:

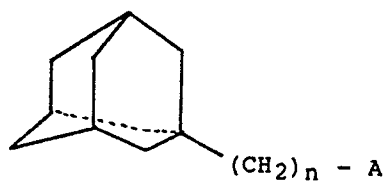

where n = 0—16, preferably 1—12, most preferably 4—8, A=COOH or $SO_3H$, and wherein the adamantane ring and the alkyl chain can be further modified and substituted with groups which are inert under the process conditions for producing branched paraffinic hydrocarbons and include $C_1$—$C_4$ alkyl groups, $NO_2$ and $CF_3$ or $C_nF_{2n+1}$ (where n = 1—10) as replacements for the remaining protons provided that at least one adamantyl bridgehead hydrogen remains to promote intermolecular hydride transfer.

Further includes are adamantyl compounds in which 2 or 3 of the 4 bridgehead protons of the adamantyl ring are replaced by a $(CH_2)_n$—COOH or $(CH_2)_n$ —$SO_3H$ chain, n varying from 0 to 16.

The alkyl chains can also contain non-reactive branches, e.g., —$(CH_2)_n$—$C(CH_3)_2$ —$(CH_2)_n$ —A, where n = (0—10), m = (0—10) and A is —$CO_2H$ or —$SO_3H$, wherein the total carbon chain is no more than 16 carbon atoms in length. The neopentyl structure existing in the above illustrated chain is non-reactive in all but the strongest acids and thus, can be used in concentrated $H_2SO_4$ or HF solutions. Expressly excluded is a single methyl group substitution, or its equivalent, which can form reactive tertiary carbonium ions in the process.

Representative examples include 16-(1′-adamantyl)hexadecanoic acid, 12-(1′-adamantyl)dodecanoic acid, 4-(1′-adamantyl)butanoic acid, 3-(1′-adamantyl)-propanoic acid, 2-(1′-adamantyl)ethanoic acid, 1′-adamantane carboxylic acid, 10-(1′-adamantyl decanoic acid, 8-(1-adamantyl)octanoic acid, 6-(1′adamantyl)hexanoic acid, 6-(2′-adamantyl)hexanoic acid, 5-(1′-adamantyl)-2-methylpentanoic acid, 5-(1′-adamantyl)pentanoic acid, 6-(1′-adamantyl)hexylsulfonic acid, 5-(1′-adamantyl)pentylsulfonic acid, 4-(1′-adamantyl)butylsulfonic acid, 4-(2′-adamantyl)butylsulfonic acid, 12-(1′-adamantyl)dodecanoic acid. A preferred catalyst compound is 6-(1′-adamantyl)hexanoic acid. It should also be noted that readily solvolyzed derivatives of these acids and their equivalents such as their esters, anhydrides, acyl halides and amides, which generate the corresponding free acid through solvolysis under "protic acid" reaction conditions, can generally be used in place of the parent adamantyl compounds and are included within the scope of the claimed process.

A further subject of this invention are new compositions of matter according to the above-given formula where A is COOH or $SO_3H$ and n is 0—16, with the proviso that when A is COOH, n is 8—16, and wherein the adamantyl ring and alkylene chain can be substituted with substituents which are inert or unreactive under process conditions for producing branched paraffinic hydrocarbons. Appropriate procedures for synthesizing the subject compositions are adequately described above.

The molar concentration of adamantyl acid in the acid solution varies from about $10^{-6}$ to $10^{-1}$ moles/liter, and preferably about $10^{-6}$ to $10^{-1}$ moles/liter. However, larger and smaller ratios can also be used effectively.

Temperatures in the process are conducted in the range of −100 to 150°C. The isomerization embodiment of the invention is carried out preferably at −50 to 100°C, depending primarily on the temperature required to obtain a liquid-phase catalyst. The paraffin-olefin alkylation embodiment of the process is carried out preferably at −10 to 30°C.

The process is normally carried out at atmospheric pressure but may also be conducted at higher pressures up to about 20 atmospheres (19.8 bars).

Yields of isomeric hydrocarbons in the process are only limited by the thermodynamic equilibrium at the process temperature, and it is within the scope of this invention to separate undesirable isomers from the mixed product and recycle them for further conversion to the more desirable isomers.

A particularly preferred embodiment of the process is where n-butane is isomerized to isobutane, n-pentane is isomerized to isopentane, and n-hexane is isomerized to a mixture of methylpentanes and dimethylbutanes.

Yields of alkylate in the process range from 150 to 204 percent of theory based on starting olefin (butylenes).

$$\text{Yield} = \frac{\text{weight of product}}{\text{weight of olefin feed}} \times 100 \quad .$$

Particularly preferred embodiments of the alkylation process are where isobutylene is reacted with isobutane to produce predominantly a mixture of 224, 234, 233 and 223 trimethylpentanes. Propylene is reacted with isobutane cation to produce a $C_7$ product comprising 2,3- and 2,4-dimethylpentanes; where isobutane is reacted with a mixture of butenes, as obtained from a petroleum feedstream, to produce a mixture comprising branched $C_8$ paraffinic hydrocarbons of which at least 80 percent are trimethylpentanes; and wherein isobutane is reacted with a mixture of amylenes, as obtained from a petroleum feedstream, to produce a mixture comprising predominantly branched $C_8$ and branched $C_9$ paraffinic hydrocarbons.

Apparatus for carrying out the present process is conventional, either on a laboratory, pilot plant, or full industrial scale and the process can be conducted in a batch-type operation or in a continuous-type operation and in liquid/liquid or liquid/gas systems. The adamantyl acid may also be used in solid/liquid or solid/gas systems, wherein its polar functionality is adsorbed onto or bound by a highly acidic solid acid. A preferred type of process is a liquid/liquid system conducted in a continuous manner.

Generally, the process is conducted by contacting a liquid mixture of paraffin and an adamantane or an amino-alkyladamantane hydrocarbon with the acid system described herein. In the paraffin-olefin alkylation embodiment of the invention, an olefin is a component of the mixture. If the hydrocarbon mixture is miscible with said acid system, then the reaction takes place in a one-phase homogeneous manner. If the acid system is, for example, $H_2SO_4$, then the process is conducted in an emulsion interface layer between the two-phases, the acid system generally being the lower phase. The entire system is preferably at reaction temperature at time of mixing during which the entire system is adequately mixed, stirred and agitated to ensure good contact between the acid system and the hydrocarbon system. The reaction is allowed to progress until a desired or substantial quantity of formed product is obtained. This can be monitored by analytical methods such as gas chromatography and mass spectrometry. After the desired paraffinic product has been formed, the phases can be separated and the hydrocarbon phase treated by extraction or fractional distillation and the like, to separate out and collect the desired product. The adamantyl carboxylic or sulfonic acid can be recovered and recycled back to the reactor for further use.

The following examples are illustrative of some modes of carrying out the invention.

## Example 1

This example illustrates the effect of surface active adamantyl carboxylic acids in accelerating intermolecular hydride transfer at a sulfuric acid/hydrocarbon interface resulting in faster isomerization of a branched paraffin, i.e., 3-methylpentane to 2-methylpentane. Listed in Table I are the relative isomerization rates of 3-methylpentane, obtained under well-stirred two-phase conditions, using equal volumes of the 3-methylpentane and 96% sulfuric acid, which contained the listed adamantyl carboxylic acids. For comparison, the isomerization rate with no adamantyl additive is also listed.

The no additive run was conducted by mixing 100 m. of conc. $H_2SO_4$, (96%) with 100 ml. of 3-methylpentane in a 500 ml., 2-neck flask at room temperature and atmospheric pressure. The two-phase system was stirred vigorously and samples of the upper hydrocarbon phase were withdrawn periodically and analyzed by gas chromatography for the extent of isomerization. The reaction was then individually repeated with 0.002 M solutions of the three listed adamantylalkyl carboxylic acids in sulfuric acid. The relative isomerization rates in the systems were measured. As seen in the data, the net isomerization rate of 3-methylpentane to 2-methylpentane more than tripled when a 0.002 M solution of any one of the adamantylalkyl carboxylic acids used, as compared to the no additive control. Also seen is the fact that increasing the carbon chain length of the alkanoic acid had a significant effect on increasing the isomerization rate.

TABLE I
Comparison of Surface Active
Hydride Transfer Catalysts in $H_2SO_4$

| Catalyst[a] | 3-Methylpentane Isomerization, Rel. Rate[b] |
|---|---|
| None (control) | 1 |
| 1′-Ad-$(CH_2)_3$COOH | 3.5 |
| 1′-Ad-$(CH_2)_4$COOH | 3.9 |
| 1′-Ad-$(CH_2)_5$COOH | 7.1 |

[a] Adamantyl catalysts used in 0.002 M concentrations, in concentrated $H_2SO_4$, 96%, at 23 ± 1°C.
[b] Relative isomerization rates based on the control run in the absence of adamantyl catalyst.

Example 2

This example illustrates the effect of 6-(1′-adamantyl)-hexanoic acid as a catalyst additive on the alkylation of 1-butene with isobutane. The runs were conducted by passing a continuous liquid mixture stream containing 90 weight percent isobutane and 10 weight percent 1-butene through 10 ml. of 98 percent sulfuric acid in a glass reactor with vigorous agitation. The temperature of the contents in the glass reactor was maintained at 10°C and the process was conducted at about 40 psig (375 kPa) pressure. In both the additive and blank runs, the feed was initially passed through the acid in order to fill the reactor within several minutes. After this time, the feed rate was adjusted and a steady state rate of 1 ml of olefin per hour and 9 ml of isobutane per hour was maintained thereafter. As the liquid hydrocarbon stream contacted the acid, an emulsion was formed between the acid, butene and butane forming a lower phase of about 20 to 30 ml. in volume and an upper phase of isobutane. As the alkylation reaction progressed, the alkylate product which was formed in the emulsified region migrated into the upper isobutane layer from which it left the glass reactor through a control valve. The volume of the upper layer was about 50 ml. The process was allowed to run for several hundred hours with the product being sampled periodically for gas chromatographic analyses, from which the alkylate selectivity (percent of $C_8$ components in the $C_5$ to first major $C_9$ component), MON (motor octane number, clear), yield and extent of cracking could be deduced. The runs using the adamantyl carboxylic acid were carried out with said adamantyl acid present in the sulfuric acid layer at a concentration of $2 \times 10^{-3}$ M. The control was run the absence of an adamantyl acid. The results are illustrated below in Table II.

The tabulated data are observations which were made at steady state conditions after an initial induction or conditioning period which lasted about 10 to 20 hours. Such periods are known and commonly observed during alkylation and are believed to relate to the buildup of reaction intermediates to a steady state concentration.

The selectivity data in Table II demonstrates that when the adamantyl additive was present, the activity maintenance of the systems was more than doubled, showing the formation of highly selective products for about 360 hours with the additive vs. 150 hours in the blank experiment. The fall-off in selectivity after these times is an indication of the acid strength decreasing to the point that efficient alkylation is no longer occurring.

The data for the unleaded motor octane number (MON, clear) show that the octane number was also slightly higher and maintained at the higher level for a longer period of time than in the blank experiment as a consequence of the increased hydride transfer rate.

The yield data, which is approximate and requiring stringent data analysis for significant conclusions, showed that the yield for the additive run was almost at the theoretical value for 550 hours on stream. The yield data for theol contained many unexplained variations but was also close to the theoretical value for only 280 hours in comparison.

The degree of cracking, which is essentially the complement weight percentage of selectivity, was substantially reduced in the additive run. As is seen, the degree of cracking in the control was never below about 7 percent, and remained in the 7 to 10 percent range for 140 hours. By contrast, the additive run exhibited only 4 to 7 percent cracking for 250 hours, before showing a 7 to 10 percent degree of cracking in the next 80 hours. At the end of the listed duration period for both runs, the degree of cracking increased significantly.

The degree of cracking in the additive case can be lowered even further by changing the startup procedure such that isobutane alone is fed into the system to fill the volume of the glass reactor up to the control valve under the conditions described in the Example, prior to the introduction of butene-1 into the system. This not only suppresses the degree of cracking, but also serves to inhibit acid-catalyzed olefin polymerization which occurs particularly with more active olefins, i.e., isobutylene.

TABLE II

Alkylation of 1-Butene

| Comparisons | $H_2SO_4$ Control | Adamantyl Additive $2 \times 10^{-3}M$ |
|---|---|---|
| o Selectivity[a] To $C_8$ isomers, % Duration, hrs.[b] | 90—92<br>150 | 90—95<br>360 |
| o MON, clear[c] Duration, hrs. | 94—95.5<br>180 | 94—96.5<br>380 |
| o Yield[d] Duration, hrs. | >150<br>280 | >150<br>550 |
| o Cracking[e] Range, %, Duration, hrs. | | |
| 4—7 | 0 | 250 |
| 7—10 | 140 | 80 |

[a] Taken as the weight percent of $C_8$ isomers in the product fraction comprising isopentane through 2,4,4-trimethylhexane and included compounds as measured on a gas chromatograph with a Supelco, SP 2110 column.

[b] Duration of run during which the selectivity was maintained in the stated range prior to significant decrease.

[c] Proprietary calculation from the gas chromatographic data used in approximating the unleaded motor fuel performance at high speed and under severe conditions.

[d] Defined as (gms. alkylate/gms. olefin feed) × 100; theoretical yield is 204 percent.

[e] Taken as the weight percent of hydrocarbons in the gas chromatographic fraction, used for determining selectivity, minus $C_8$ isomers.

**Claims**

1. A process for producing branched paraffinic hydrocarbons wherein a $C_4$—$C_6$ paraffinic compound is contacted in a reaction zone with a strong acid system and a reagent selected from an adamantyl carboxylic acid containing at least one unsubstituted bridgehead position and an adamantyl sulfonic acid containing at least one unsubstituted bridgehead position, said contacting being carried out at a temperature of from −100 to 150°C.

2. A process according to claim 1 wherein said process is an alkylation process, said $C_4$—$C_6$ paraffinic compound is a linear or branched paraffinic compound capable of forming a carbonium ion under strong acid conditions and said process is carried out in the additional presence of a $C_2$—$C_5$ olefin to produce a $C_6$—$C_{11}$ branched paraffinic hydrocarbon.

3. A process according to claim 2 wherein said paraffinic compound is selected from n-butane, isobutane, n-pentane, isopentane, n-hexane, 2-methylpentane, isomers thereof and mixtures thereof.

4. A process according to claim 2 or claim 3 wherein said olefin is selected from ethylene, propylene, butene-1, cis or trans butene-2, isobutylene, pentenes, isomers thereof and mixtures thereof.

5. A process according to claim 1 wherein said process is an isomerization process, said $C_4$—$C_6$ paraffinic compound is a non-cyclic paraffinic hydrocarbon selected from n-butane, n-pentane, n-hexane, 2-methylpentane, 3-methylpentane, isomers thereof and mixtures thereof.

6. A process according to any one of claims 1—5 wherein said acid system contains an acid component selected from $AlBr_3$, $AlCl_3$, $GaCl_3$, $TaF_5$, $SbF_5$, $AsF_5$, $BF_3$, HF, HCl, HBr, $H_2SO_4$, $HSO_3F$, $CF_3SO_3H$ and mixtures thereof.

7. A process according to any one of claims 1—6 wherein said acid system further contains a solvent selected from $CH_3Br$, $CH_2Br_2$,$CH_2Cl_2$, 1,2-dichloroethane, 1,2,3-trichlorobenzene, 1,2,3,4 tetrachlorobenzene, pentafluorobenzene and mixtures thereof.

8. A process according to any one of claims 1—7 wherein said adamantyl carboxylic acid is 6-(1′-adamantyl)hexanoic acid.

9. An adamantyl carboxylic acid or sulfonic acid composition of the formula:

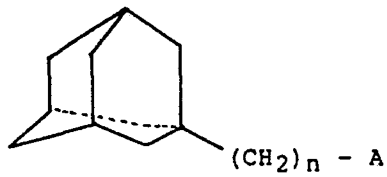

wherein A is COOH or $SO_3H$, and n is 0—16, with the proviso that where A is COOH, n is 8—16, and wherein the adamantyl ring and alkylene chain can be substituted with substituents which are inert or unreactive under process conditions for producing branched paraffinic hydrocarbons.

10. A process according to any one of claims 1 to 7 wherein said adamantyl carboxylic acid or adamantyl sulphonic acid is that claimed in claim 9.

**Patentansprüche**

1. Verfahren zur Herstellung von verzweigten paraffinischen Kohlenwasserstoffen, dadurch gekennzeichnet, daß eine paraffinische $C_4$—$C_6$ Verbindung in einer Reaktionszone mit einem starken Säuresystem und einem Reagenz ausgewählt aus einer Adamantylcarbonsäure mit mindestens einer nicht substituierten Brückenkopfposition und einer Adamantylsulfonsäure mit mindestens einer nicht substituierten Brückenkopfposition bei einer Temperatur von −100 bis 150°C kontaktiert wird.

2. Verfahren nach Anspruch 1, dadurch gekennzeichnet, daß das Verfahren ein Alkylierungsverfahren ist, die paraffinische $C_4$—$C_6$ Verbindung eine linerare oder verzweigte paraffinische Verbindung ist, die unter stark sauren Bedingungen ein Carboniumion bilden kann, und das Verfahren in der zusätzlichen Gegenwart eines $C_2$—$C_5$ Olefins durchgeführt wird, um einen verzweigten paraffinischen $C_6$—$C_{11}$ Kohlenwasserstoff herzustellen.

3. Verfahren nach Anspruch 2, dadurch gekennzeichnet, daß die paraffinische Verbindung ausgewählt ist aus n-Butan, Isobutan, n-Pentan, Isopentan, n-Hexan, 2-Methylpentan, Isomeren derselben und Mischungen derselben.

4. Verfahren nach Anspruch 2 oder 3, dadurch gekennzeichnet, daß das Olefin ausgewählt ist aus Ethylen, Propylen, Buten-1, Cis- oder Trans-Buten-2, Isobutylen, Pentenen, Isomeren derselben und Mischungen derselben.

5. Verfahren nach Anspruch 1, dadurch gekennzeichnet, daß es ein Isomerisierungsverfahren ist und die paraffinische $C_4$—$C_6$ Verbindung ein nicht-cyclischer paraffinischer Kohlenwasserstoff ausgewählt aus n-Butan, n-Pentan, n-Hexan, 2-Methylpentan, 3-Methylpentan, Isomeren derselben und Mischungen derselben ist.

6. Verfahren nach einem der Ansprüche 1 bis 5, dadurch gekennzeichnet, daß das Säuresystem eine Säurekomponente ausgewählt aus $AlBr_3$, $AlCl_3$, $GaCl_3$, $TaF_5$, $SbF_5$, $AsF_5$, $BF_3$, HF, HCL, HBR, $H_2SO_4$, $CF_3SO_3H$ und Mischungen derselben enthält.

7. Verfahren nach einem der Ansprüche 1 bis 6, dadurch gekennzeichnet, daß das Säuresystem außerdem ein Lösungsmittel ausgewählt aus $CH_3Br$, $CH_3Br_2$, $CH_2Cl_2$, 1,2-Dichlorethan, 1,2,3-Trichlorbenzol, 1,2,3,4-Tetrachlorbenzol, Pentafluorbenzol und Mischungen derselben enthält.

8. Verfahren nach einem der Ansprüche 1 bis 7, dadurch gekennzeichnet, daß die Adamantylcarbonsäure 6-(1′-Adamantyl)capronsäure ist.

9. Adamantylcarbonsäure- oder -sulfonsäurezusammensetzung mit der Form 1:

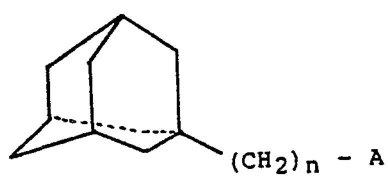

in der A COOH oder $SO_3H$ und n 0—16 mit der Maßgabe sind, daß n 8—16 ist, wenn A COOH ist, und wobei der Adamantylring und die Alkylenkette mit Substituenten substituiert sein können die unter Verfahrensbedingungen für die Herstellung von verzweigten paraffinischen Kohlenwasserstoffen inert oder nicht reaktiv sind.

10. Verfahren nach einem der Ansprüche 1 bis 7, dadurch gekennzeichnet, daß die Adamantylcarbonsäure oder Adamantylsulfonsäure diejenige gemäß Anspruch 9 ist.

**Revendications**

1. Procédé pour produire des hydrocarbures paraffiniques ramifiés, dans lequel un composé paraffinique en $C_4$—$C_6$ est mis en contact, dans une zone de réaction, avec un système acide fort et un réactif choisi parmi un acide adamantylcarboxylique contenant au moins une position en tête de pont non substituée et un acide adamantylsulfonique contenant au moins une position en tête de pont non substituée, cette mise en contact étant réalisée à une température de −100 à 150°C.

2. Procédé selon la revendication 1, dans lequel le procédé est un procédé d'alkylation, le composé paraffinique en $C_4$—$C_6$ est un composé paraffinique linéaire ou ramifié capable de former un ion carbonium dans des conditions fortement acides et le procédé est réalisé en présence additionnelle d'une oléfine en $C_2$—$C_5$ pour produire un hydrocarbure paraffinique ramifié en $C_6$—$C_{11}$.

3. Procédé selon la revendication 2, dans lequel le composé paraffinique est choisi parmi le n-butane, l'isobutane, le n-pentane, l'isopentane, le n-hexane, le 2-méthylpentane, les isomères de ceux-ci et les mélanges de ceux-ci.

4. Procédé selon la revendication 2 ou la revendication 3, dans lequel l'oléfine est choisie parmi l'éthylène, le propylène, le butène-1, le butène-2 cis ou trans, l'isobutylène, les pentènes, les isomères de ceux-ci et les mélanges de ceux-ci.

5. Procédé selon la revendication 1, dans lequel ce procédé est un procédé d'isomérisation, le composé paraffinique en $C_4$—$C_6$ est un hydrocarbure paraffinique non cyclique choisi parmi le n-butane, le n-pentane, le n-hexane, le 2-méthylpentane, le 3-méthylpentane, les isomères de ceux-ci et les mélanges de ceux-ci.

6. Procédé selon l'une quelconque des revendications 1 à 5, dans lequel le système acide contient un constituant acide choisi parmi $AlBr_3$, $AlCl_3$, $GaCl_3$, $TaF_5$, $SbF_5$, $AsF_5$, $BF_3$, HF, HCl, HBr, $H_2SO_4$, $HSO_3F$, $CF_3SO_3H$ et les mélanges de ceux-ci.

7. Procédé selon l'une quelconque des revendications 1 à 6, dans lequel le système acide contient en outre un solvant choisi parmi $CH_3Br$, $CH_2Br_2$, $CH_2Cl_2$, le 1,2-dichloroéthane, le 1,2,3-trichlorobenzène, le 1,2,3,4-tétrachlorobenzène, pentafluorobenzène et les mélanges de ceux-ci.

8. Procédé selon l'une quelconque des revendications 1 à 7, dans lequel l'acide adamantylcarboxylique est l'acide 6-(1'-adamantyl)hexanoïque.

9. Composition d'acide adamantylcarboxylique ou sulfonique de formule:

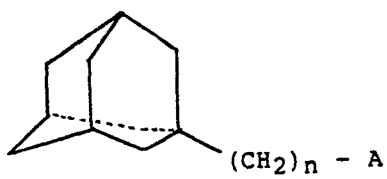

dans laquelle A est COOH ou $SO_3H$, et n est un nombre de 0 à 16, à condition que lorsque A est COOH, n soit est un nombre de 8 à 16, et dans laquelle le cycle adamantyle et la chaîne alkylène peuvent être substitués par des substituants qui sont inertes ou non réactifs dans les conditions du procédé pour la production d'hydrocarbures paraffiniques ramifiés.

10. Procédé selon l'une quelconque des revendications 1 à 7, dans lequel l'acide adamantylcarboxylique ou l'acide adamantylsulfonique est celui qui est revendiqué dans la revendication 9.